Europäisches Patentamt

European Patent Office

Office européen des brevets

(11) Publication number: **0 427 189 A1**

# EUROPEAN PATENT APPLICATION

(21) Application number: 90121187.0

(51) Int. Cl.5: **C07K 13/00, C12N 15/16**

(22) Date of filing: 06.11.90

(30) Priority: 07.11.89 JP 289143/89

(43) Date of publication of application:
15.05.91 Bulletin 91/20

(84) Designated Contracting States:
AT BE CH DE FR GB IT LI NL SE

(71) Applicant: SNOW BRAND MILK PRODUCTS & CO., LTD.
1-1, Naebo-cho 6-chome Higashi-ku Sapporo-shi Hokkaido 065(JP)

(72) Inventor: Akai, Kunihisa
2-880-12 Kahinata Satte
Saitama(JP)
Inventor: Yamaguchi, Kyoji, 1-524 Lions Garden
Higashi Omiya, 702-12 Shima-cho
Omiya, Saitama(JP)
Inventor: Ueda, Masatsugu
719 Mezon Musashino, 1672-1 Imafuku
Kawagoe, Saitama(JP)

(74) Representative: Boeters, Hans Dietrich, Dr. et al
Boeters & Bauer Bereiteranger 15
W-8000 München 90(DE)

(54) **Modified forms of human erythropoietin and DNA sequences encoding genes which can express them.**

(57) A new potent modified human erythropoietin in whicht least one of two Asn residues at the 24th and 83th from N-terminal is omitted or substituted by amino acids other than Asn and a DNA sequence which express it.

```
                                                    -27
                                                    Met Gly Val His Glu Cys Pro

-20                                       -10                                    -1
Ala Trp Leu Trp Leu Leu Leu Ser Leu Leu Ser Leu Pro Leu Gly Leu Pro Val Leu Gly

1                      SH        10                                           20
Ala Pro Pro Arg Leu Ile Cys Asp Ser Arg Val Leu Glu Arg Tyr Leu Leu Glu Ala Lys

            *                    SH  30        . SH              *            40
Glu Ala Glu Asn Ile Thr Thr Gly Cys Ala Glu His Cys Ser Leu Asn Glu Asn Ile Thr

                                 50                                           60
Val Pro Asp Thr Lys Val Asn Phe Tyr Ala Trp Lys Arg Met Glu Val Gly Gln Gln Ala

                                 70                                           80
Val Glu Val Trp Gln Gly Leu Ala Leu Leu Ser Glu Ala Val Leu Arg Gly Gln Ala Leu

        *                        90                                           100
Leu Val Asn Ser Ser Gln Pro Trp Glu Pro Leu Gln Leu His Val Asp Lys Ala Val Ser

                                 110                                          120
Gly Leu Arg Ser Leu Thr Thr Leu Leu Arg Ala Leu Gly Ala Gln Lys Glu Ala Ile Ser

            (*)                  130                                          140
Pro Pro Asp Ala Ala Ser Ala Ala Pro Leu Arg Thr Ile Thr Ala Asp Thr Phe Arg Lys

                                 150                                          160
Leu Phe Arg Val Tyr Ser Asn Phe Leu Arg Gly Lys Leu Lys Leu Tyr Thr Gly Glu Ala

SH                   166
Cys Arg Thr Gly Asp(Arg)
```

Fig. 1

# MODIFIED FORMS OF HUMAN ERYTHROPOIETIN AND DNA SEQUENCES ENCODING GENES WHICH CAN EXPRESS THEM.

(2) Field of the Invention

The present invention relates to novel modified forms of human erythropoietin and DNA sequences encoding genes which can express them.

(3) Description of the Prior Art

Erythropoietin (hereafter referred to as EPO) is a hemopoietic factor which is produced in the kidney and acts to progenitor cells of erythrocytes to stimulate their proliferation and differentiation to erythrocytes Therefore, it is well known that EPO is very effective for the treatment of anemia.

A small amount of EPO is extracted in human urine, especially a fairly high concentration of EPO is contained in urine of patients of aplastic aneamia. This material has been isolated and used as a reagent for laboratory use. On the other hand, a certain amount of EPO can be recently produced by development of the gene technology which enables to produce a large amount of the physiologically active proteins, using the gene encoding EPO ( Jacobs et al., Nature vol. 313, 806-810, 1985 ). Also, the method about the production of EPO using the gene technology has been presented in Tokuhyoushou 61-501152, Tokuhyoushou 61-501627, Tokuhyoushou 62-501010, Japanese Laid Open No.62-269697, Japanese Laid Open No.62-19838, Japanese Laid Open No.63-126488, and Japanese Laid Open No.62-171696.

EPO is a heavily glycosylated protein, which has a molecular weight of 34,000-39,000, and it is well-known that a half of the molecular weight is consisted of the sugar chain. It is also said that existence of the sugar chains important to express a in vivo biological activity of EPO. Goto et al. (Goto et al., Biotechnology vol. 6, 67-71, 1988) have demonstrated that the sugar chain is varied dependingly upon host cells when EPO is produced by the gene technology. Thus, it is not represented in the precious published official bulletins that the sugar chain structure of EPO is varied through its production using the gene technology.

The human EPO which is produced by the gene technology has 3 binding sites of N-glycoside type sugar chain and 1 binding site of O-glycoside type sugar chain.

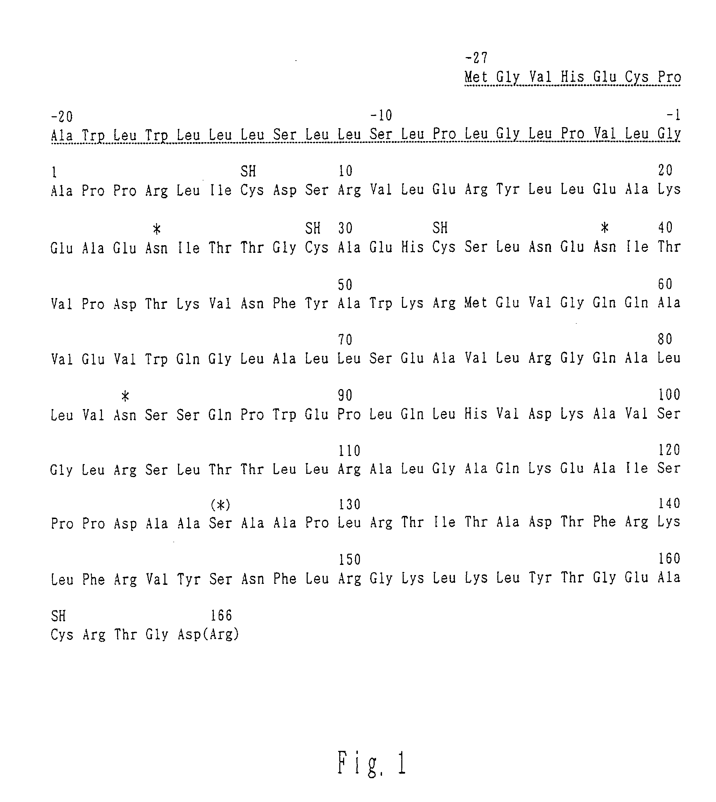

It is known that Asn indicated as * and Ser indicated as (*) in Fig.1 are thecorresponding binding sites, respectively.

Sialic acid is bound to the non-reduced terminal of EPO sugar chain. It is also well-known that removal of the sialic acid induces an increase in the affinity of EPO to its receptor which is located on the surface of target cells (bone marrow cell CFU-E), and it results in an enhancement of EPO activity. However, such EPO has a disadvantage that it is rapidly metabolized and therefore has less activity in vivo, because terminal galactose residue is exposed on EPO molecule by the removal of sialic acid and thus EPO is captured by the receptor which exists in the liver and binds specifically to exposed galactose residue. Therefore, it is necessary to make an EPO which has a high affinity to the EPO receptor existing on the surface membrane of target cells and in which galactose residue is not exposed at the non-reduced terminal of sugar chain for supplying an EPO with the higher activity as a remedy for treatment of anemia. Thus EPO in which the sugar chain structure is varied and its production method have not been reported yet untill the point of submitting the present invention.

As described above, although it is known that the biological activity of EPO is changed by altering the sugar chain structure, such EPO in which the sugar chain structure is specified has not been obtained yet untill the point of submitting the present invention.

Thereafter, a modified EPO in which Lys residue is substituted by homoarginine and guanidylated (Japanese Laid Open No.2-9900) and a modified EPO in which Met residue at the 54th from N-terminal is substituted by Leu, or Asn residue at the 38th is substituted by Gln (Japanese Laid Open No.2-59599) have been reported. Furthermore, various hormones including EPO with the biological activity in which Lys residue is removed or substituted by other amino acids(Tokuhyouhei 2-502646).

(4) Summary of the Invention

The present inventor has found that an EPO in which the number of sialic acid residue existing at the non-reduced terminal of sugar chain in native EPO is reduced and galactose residue is not exposed can be obtained by substituting the amino acid residue at the binding site of sugar chain in EPO by other amino acids.

Therefore, an object of the present invention is to offer the new modified EPO which has a unique primary structure. Also, another object of the present invention is to offer the new modified EPO which is not easy captured by the receptor which bind specificaly galactose and which has a high affinity to the EPO receptor existing on the surface membrane of target cells. Furethermore, the object of the present invention is to offer the new modified EPO which has a higher biologicalactivity than that of native EPO. Also, another object of the present invention is to offer DNA encoding thegene for the expression of such modified EPO.

The objects of the present invention are accomplished by supplying the modified EPO in which at least one of Asn residues at the 24th and 83th or both two and Asn residue at 38th are omitted, or these residues are substituted by amino acids other than Asn.

In order to obtain the modified EPO according to the present invention, DNA which can express the modified EPO is constructed the modified EPO is produced by the method using gene technology.

(5)Brief Description of the Drawings

Fig.1 shows a full sequence of amino acid arrangement of EPO. Asn indicated as * in the Figure is omitted or substituted by other amino acids in the modified EPO in the present invention.

Fig.2 shows the structure of the primer which is used in vitro site specific mutagenesis of EPO gene.

Fig.3 shows a construction of the vector which has a modified arrangement of EPO gene.

Fig.4 shows a construction of the expression vector of the modified EPO [ pZIP NeoSV(X)1-EPO(m) ].

(6)Detailed Description of Prefer Embodiments

At first it is necessary to obtain a gene encoding EPO in order to get the modified EPO in the present invention. There are two kinds of gene, cDNA which does not have a intron and chromosomal DNA which has an intron, and both DNA can be used in the present invention. The above cDNA can be obtained by cloning according to the method described in Tokuhyoushou 62-501010. Chromosomal DNA can be obtained according to the method of Goto et al. (Goto et al ., Biotechnology vol.6, 67-71, 1988). They can also be obtained by the direct cloning of human chromosomal DNA bank according to the common method. This DNA encoding EPO is cloned to a phage vector, M13mp19 (Takara shuzo), and then DNA sequences corresponding to the 24th and/or the 83th, or the 38th amino acids in addition to the both, if necessary, from N-terminal of the EPO gene is subjected to a site specific in vitro mutagenesis. Omission of the corresponding amino acids is carried out as follows; an at least 17 mer oligonucleotide is synthesized near the center of the nucleotide sequence which is deficient of the corresponding amino acids, and this is employed as a primer using a site-specific in vitro mutagenesis kit on the market. A kit from Amersham on the market can be used as the above kit.

In the case of substitution of the corresponding amino acids by other amino acids, the similar site -specific in vitro mutagenesis can be carried out through the synthesis of a primer in which the codon corresponding to the amino acids is substituted by the codon of the substituted amino acids. Although Asn is substituted by Gln in order to diminish the effect on the structure of the protein in the present invention, several amino acids can be used for substitution according to the purpose.

M13mp19 which is in vitro mutagenized as above is transfected into Escherichia coli NM522, and the modified gene is sujected to screening following the detection of a phage plaque which has the aimed modified amino acid sequences by analysis of DNA sequences.

The aimed gene which has the modified nucleotide sequence is obtained as follows; the above obtained gene is cloven with restriction enzymes BamH I -Bgl II , and isolated. The obtained gene is inserted into the site cleavaged with a restriction enzyme BamH I , down stream of LTR and upstream of Neo gene of mammalian cell shuttle vector pZIP-NeoSV(X)1. The gene in which EPO gene is inserted correctly is selected by mapping analysis of restriction enzyme cleavage. Thus obtained vector with the modified EPO gene is called pZIP-NeoSV (X)1-EPO(m). pZIP-NeoSV(X)1 is the shuttle vector which Cepko et al. (Cepko et al., Cell vol. 37, 1053-1062, 1984) have developed and can be obtained by their method.

Thus obtained plasmid which has the modified EPO gene is transfected into the mammalian cells and the cells are cultured. The secreted EPO is obtained in the supernatant.

The present invention is further described in detail as shown in EXAMPLES in the following.

EXAMPLE

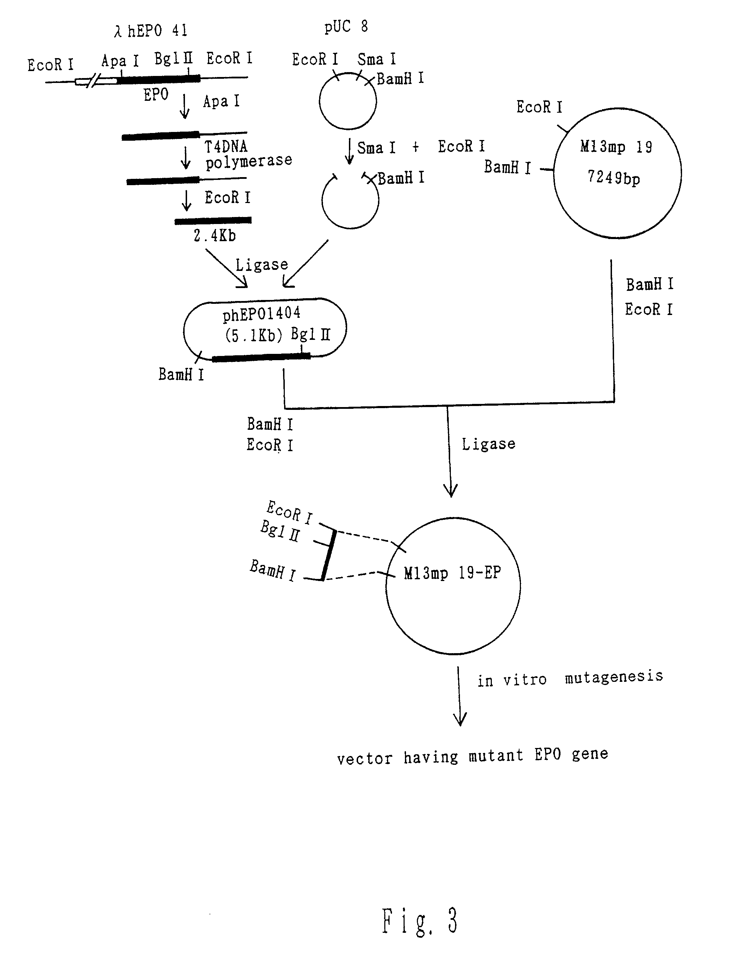

①Cloning of EPO gene(see Fig.3).

Human EPO gene was cloned according to the method of Goto et al. (Goto et al., Biotechnology vol. 6, 67-71, 1988).

A gene containing Apa I site and Bgl II site of EPO gene which was cloned from human genomic library of chromosomal DNA of human fetal liver was inserted into the cleavage sites of Sma I and EcoR I of the plasmid vector pUC8 and a vector phEPO1404 which has EPO gene was obtained.

②Cloning of EPO gene to M13mp19(see Fig.3).

After 10 μl of 10-fold concentration of EcoR I reaction buffer (1M Tris-HCl,pH7.5. 70mM MgCl$_2$, 500mM NaCl, 70mM 2-mercaptoethanol, 0.1% bovine serum albumin) was added to 13.4 μg of the plasmid phEP1404 (DNA segment of 2.4 Kb downstream from the Apa I -cleavage site of 5′-terminal of EPO gene was inserted into the cleavage site of the plasmid vector pUC8 with the restriction enzymes Sma I and EcoR I dissolved in 90 μl of water,20 units of the restriction enzyme EcoR I was added to the mixture. The reaction was carried out at 37°C for 2 hours. After termination of the reaction, DNA was collected by ethanol precipitation and dissolved in 90μl of water. After addition of 10μl of BamH I reaction buffer ( 100 mM Tris-HCl, pH8.0, 70mM MgCl$_2$, 1M NaCl, 20mM 2-mercaptoethanol, 0.1% bovine serum albumin ) to this, 20 units of the restriction enzyme EcoR I was added and the reaction was carried out at 37°C for 2 hours. After termination of the reaction, electrophoresis using 3.5% polyacrylamide gel was performed and a part of the gel corresponding to 2.4 Kb EcoR I -BamH I segments (EPO gene) was cut down. The contained DNA segments were eluted electrophoretically, precipitated with ethanol, dissolved in 20μl of water, and used as the EPO gene solution.

On the other hand, after 10 μg of the phage vector M13mp19 dissolved in 90 μl of water was treated with EcoR I and BamH I similarly to the above, it was precipitated with ethanol, dissolved in 20μl of water and used as the vector solution. 8μl of the EPO gene solution and 1μl of the vector solution were mixed, 72μl of A solution and 9 μl of B solution of a DNA ligation kit ( Takara Shuzou ) were added, and the reaction was carried out at 16°C for 30 min. Escherichia coli NM522 competent cells were transformed with 5 μl of the reaction mixture using soft agar containing 1% IPTG and 1% Xgal. Double strand phage DNA was prepared from the colorless plaque among those which were produced, and M13mp19-EP in which the aimed EPO gene was inserted into M13mp19 at the cleavage site of EcoR I - BamH I was selected by the analysis of restriction enzyme cleavage mapping. Single strand phage DNA was prepared and used as a template for site- specific in vitro mutagenesis.

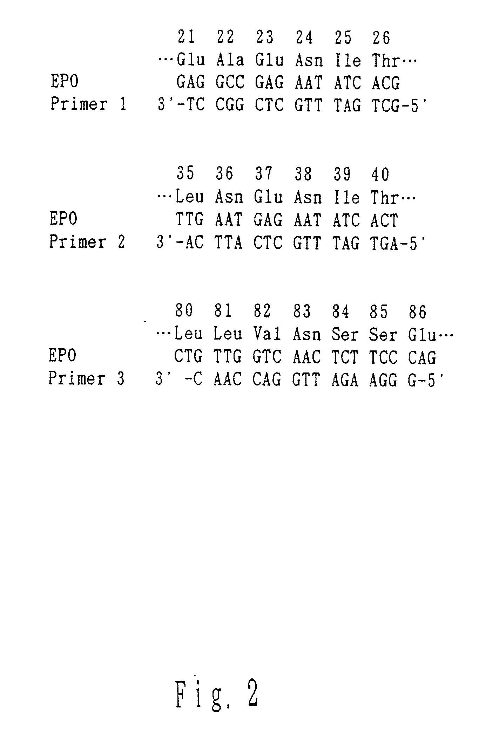

③Site-specific in vitro mutahgenesis of EPO gene(M13mp19-EP) (see Fig.2).

Site-specific mutagenesis was carried out according to the method that Asn codon of recognition sequence Asn-X-(Ser or Thr) of N-bound sugar chain binding site is substituted by Gln codon to which sugar chain is not bound.

Three oligonucleotides shown in Fig.2 were prepared using an automatic DNA synthesizer of Applied Biosystems, and used as primers for site-specific in vitro mutagenesis by which codons corresponding to the 24th, 38th and 83th Asn residues were substituted by Gln codon, respectively.

Site-specific in vitro mutagenesis was carried out using site-specific in vitro mutagenesis system with oligonucleotides of Amersham.

After the reaction, Escherichia coli NM522 was transformed. Single strand DNA was prepared from the occured plaques and the aimed plaque was selected by DNA sequence analysis according to the dideoxy method of Sanger et al.. Thus, phage vectors NQ-1., NQ-2 and NQ-3 in which codons corresponding to the 24th, 38th and 83th Asn residues were substituted by Gln codon were obtained. Then, in vitro mutagenesis that codon corresponding to the 38th or 83th Asn residue was substituted by Gln codon using phage vector NQ-1 as a template and in vitro mutagenesis that codon corresponding to 83th Asn residue was substituted by Gln codon using phage vector NQ-2 as a template were carried out, respectively, and phage vectors NQ-12, Nq-13 and NQ-23 were obtained. Furthermore, in vitro mutagenesis that codon corresponding to the 83th Asn residue was substituted using phage vector NQ-12 as a template was carried out and phage vector NQ-123 was obtained.

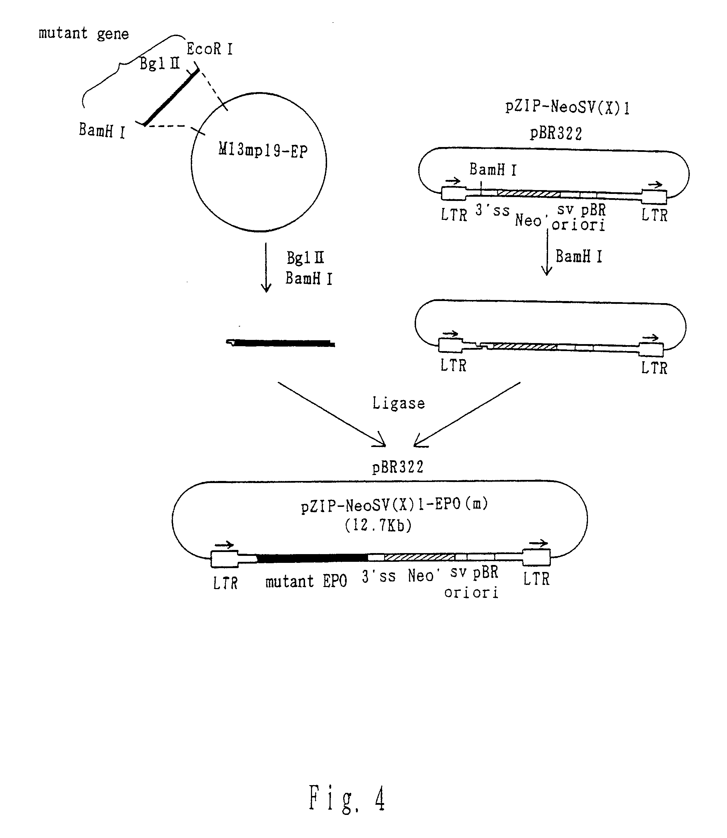

④Preparation of expression vector for the modified EPO gene(see Fig.4)

Each 10μg of 7 modified phage vectors obtained as described above was dissolved in 360μl of water. After addition of 40μl of 10-fold concentration of Bgl II reaction buffer (1M Tris-HCl, pH7.5, 70mM $MgCl_2$, 1M NaCl, 70 mM 2-mercaptoethanol, 0.1% bovine serum albumin) to the solution, each 20 units of BamH I and Bgl II was added and the reaction was carried out at 37°C for 2 hours. After termination of the reaction,electrophoresis using 3.5% polyacrylamid gel was performed and a band corresponding to 2.4 Kb was cut down.The contained DNA segments were eluted electrophoretically, precipitated with ethanol, and dissolved in 20 μl of water.

On the other hand, after 10μg of the expression vector pZIPNeoSV(X)1 was dissolved in 90 μl of water, 10μl of 10-fold concentration of Bam H I reaction buffer and 20 units of BamH I were added and the reaction was carried out at 37°C for 2 hours. Purification was carried out similarly to the above and the vector solution was obtained.

After mixing of 8μl of the modified EPO gene solution with 1μl of the vector solution, ligation was carried out as descrived above, and 50μl of Escherichia coli HB 101 competent cells were transformed with 5μl of the reaction mixture.

Seven strains of Escherichia coli which have the aimed transfection vector for the modified EPO were selected by the analysis of plasmid of restriction enzyme cleavage mapping in strains obtained from ampicilin-kanamycin resistant transformant according to the above procedures. In these Escherichia coli, the strains inserted NQ-1 or NQ-3 were deposited to the Fermentation Research Institute as **FERM BP-3 149 and FERM BP.3 150** respectively.

Plasmid was prepared by the common method from these Escherichia coli and the modified EPO gene was introduced into BHK cells (baby syrian hamster kidney cell) according to the DNA transfection method as follows.

⑤Introduction of expression vector for the modified EPO gene into mammalian cells.

After 1 day culture of $4 \times 10^5$ BHK cells in 6 cm O dish, DNA transfection was carried out according to the calcium phosphate method. 25μg of the expression vector of the modified EPO gene dissolved in 25μl of water, 75μl of 2M KCl, and 525μl of water were mixed and the mixture was used as A solution. Then, 625μl of 50 mM HEPES(pH7.1) containing 0.28M NaCl and 12.5 μl of 35mM $NaH_2PO_4$ and 35mM $NaHPO_4$ were mixed and used as B solution. A solution was added slowly dropwise into B solution with vigorously stirring. DNA was co-precipitated with calcium phosphate and precipitationwas completed by standing at room temperature for 30 min.

The DNA-calcium phosphate solution (0.4ml) prepared as descrived above was added to 4 ml of BHK cell culture { Basal Medium Eagle ( BME ) + 10% CS + 10% Tryptase Phosphate Broth } prepared previously and the cells were cultured in a $CO_2$ incubator for 18 hours.

0.4ml of PBS solution containing 25% glycerol was added to the obtained culture. The cells were stand for 2 min, and washed with BME solution 3 times. After addition of 4 ml of the above culture broth, the cells were further cultured in a $CO_2$ incubator. After 1 day culture, they were harvasted and inoculated again in 5 dishes (1/5 split) and the medium was changed to that containing 400μg/ml G418 next day. Then, the culture was continued for 2 weeks with occasional medium change and the colony of G418 resistant cells was obtained.

The culture broth of the dish in which the colony was grown, was removed and the cells were washed with PBS. A small piece of filter soaked with 25 μg/ml trypsin was put on the colony in the dishe. After 1~2 min, the cells attached to the filter were collected and cultured in 24-well dishes. Activity of EPO of the cultured supernatant in each well was measured by the enzyme immunoassay ( EIA ) method and the cell producing potent EPO activity was selected.

⑥Production of the modified EPO and preparation of partially purified material.

The cells ($1.5 \times 10^5$) obtained as descrived above in which each 7 modified EPO gene was introduced were innoculated into 4 T-flask (170 $cm^2$) and cultured for 3 days in a medium (BME + 10% CS + 10%

5

TPB) followed by the change to a medium (BME + 2% CS). Then, the cultured supernatant was collected every 3 days.

Each 1l of the obtained cultured supernatant was concentrated to by ultrafiltration equipment with a molecular weight less than 13,000 through the ultra filtration keeping under temperature control (5-10° C). The concentrated fraction was de-salted by addition of water and freeze-dried. The dried powder was dissolved in a small volume of PBS (phosphate buffer saline) and dialyzed against a large volume of PBS. The dialyzed inner solution was centrifuged and the precipitation was removed. The supernatant was applied to an antibody-absorption column which was prepared by absorption of a monoclonal antibody against human EPO to Affi-gel 10 (antibody absorption amount: 15mg/ml Affi-Gel 10; 1.3cm × 1.5cm. bed volume 2ml). Then, after the column was washed with 150 ml of PBS, 30 ml of 10mM phosphate buffer (pH7.4) containing 0.5M NaCl, and then 30ml of 0.15M NaCl, the modified EPO was eluted with the mixture of 0.2M acetic acid and 0.15M NaCl. Absorption at 280 nm of each fraction was determined and the eluted fraction was collected. The fraction was neutralized by the addition of an adequate volume of 3.4M Tris solution and concentrated using Centricutmini ( Kurabo, fractionated molecular weight 10,000).

Yield and recovery of each modified EPO are shown in Table 1.

Table 1

| Modified EPO | Yield(U) | Recovery(%) |
|---|---|---|
| NQ1 | 11730 | 45 |
| NQ2 | 4617 | 45 |
| NQ3 | 7475 | 55 |
| NQ12 | 7540 | 67 |
| NQ13 | 2210 | 54 |
| NQ23 | 605 | 15 |
| NQ123 | 380 | 18 |

⑦Biological activity of the modified EPO

The activity of the modified EPO obtained as described above was determined by in vitro bioassay of MTT assay using EPO dependent cell line EP-EDC-P2 which requires EPO for the growth and the survival. Table 2 shows the concentration of each modified EPO which exhibited 1/2 of the maximal stimulation of the cell growth.

Table 2

| Modified EPO | 1/2 max concentration (mv/ml) |
|---|---|
| Native | 23.5 |
| NQ1$^{23Asn}$ | 9.5 |
| NQ2$^{38Asn}$ | 9.8 |
| NQ3$^{83Asn}$ | 10.6 |
| NQ12$^{24, 83Asn}$ | 8.5 |
| NQ23$^{38, 83Asn}$ | 24.0 |
| NQ123$^{24, 38, 83Asn}$ | 16.0 |

In the table 24 Asn, 38Asn and 83Asn indicate the modified EPO in which Asn residues at the 24th, 38th and 83th were substituted by Gln, respectively. Also, 24, 38 Asn, 24, 83 Asn, 38,83Asn and 24, 38, 83 Asn indicate the modified EPO in which Asn residues at the 24th and 38th, at the 24th and 83th, at the 38th and 83th, and at the 24th, 38th and 83th, were substituted by Glu respectively.

As described above, the most part of the modified EPO exhibited higher activity than native recombinant EPO. Especialy, NQ13 showed 4 fold increase of the activity.

Table 3

| modified EPO | Relative activity (in vivo/EIA) |
|---|---|
| Native | 100 |
| NQ1 | 139 |
| NQ2 | 61 |
| NQ3 | 105 |
| NQ12 | 0 |
| NQ13 | 42 |
| NQ23 | 57 |
| NQ123 | 26 |

As described in Table 3, the relative activity of NQ1 has increased 1.4 fold by comparison with native EPO.

## Claims

(1)A modified human erythropoietin which is characterized by that at least one of two Asn residues at the 24th and 83th from N-terminal in its amino acid sequence is omitted or substituted by amino acids other than Asn.

(2)A modified human erythropoietin according to claim(1), in which Asn residue at the 24th from N-terminal is substituted by Gln.

(3)A modified human erythropoietin according to claim(1), in which Asn residues at the 24th and 83th from N-terminal are substituted by Gln.

(4)A modified human erythropoietin which is characterized by that at least one of two Asn residues at the 24th and 83th and another Asn residue at the 38th from N-terminal in its amino acid sequence are omitted or substituted by amino acids other than Asn.

(5)A modified human erythropoietin according to claim(4), in which Asn residues at the 24th and 38th from N-terminal are substituted by Gln

(6)A DNA sequence encoding gene which express the modified human erythropoietin in which at least one of two Asn residues at the 24th and 83th from N-terminal is omitted or subsituted by amino acids other than Asn.

(7)A DNA sequence encoding gene which express the modified human erythropoietin in which at least one of two Asn residues at the 24th and 83th and another Asn residue at the 38th from N-terminal are omitted or substituted by amino acids other than Asn.

(8)A DNA sequence encoding gene according to claim(6) or (7), which express the modified human erythropoietin in which the substituted amino acid is Gln.

(9) A Escherichia coli strain, which is capable of expressing the modified human erythropoietin of claim(1) or claim(4).

(10) The Esherichia coli strain as claimed in claim(9) which was deposited to the Fermentation Research Institute as the deposit number **FERM BP-3 149.**

(11) The Escherichia coli strain as claimed in claim (9) which was deposited to the Fermentation Research Institue as the deposit number **FERM BP-3 150.**

## Sequence Listing

(1)

```
                 -27
                 Met Gly Val His Glu Cys Pro

 -20
 Ala Trp Leu Trp Leu Leu Leu Ser Leu Leu

 -10                                     -1
 Ser Leu Pro Leu Gly Leu Pro Val Leu Gly

 1                          SH          10
 Ala Pro Pro Arg Leu Ile Cys Asp Ser Arg

                                        20
 Val Leu Glu Arg Tyr Leu Leu Glu Ala Lys

                 *                  SH  30
 Glu Ala Glu Asn Ile Thr Thr Gly Cys Ala

         SH                     *       40
 Glu His Cys Ser Leu Asn Glu Asn Ile Thr

                                        50
 Val Pro Asp Thr Lys Val Asn Phe Tyr Ala

                                        60
 Trp Lys Arg Met Glu Val Gly Gln Gln Ala

                                        70
 Val Glu Val Trp Gln Gly Leu Ala Leu Leu

                                        80
 Ser Glu Ala Val Leu Arg Gly Gln Ala Leu

         *                          90
 Leu Val Asn Ser Ser Gln Pro Trp Glu Pro

                                        100
 Leu Gln Leu His Val Asp Lys Ala Val Ser

                                        110
 Gly Leu Arg Ser Leu Thr Thr Leu Leu Arg

                                        120
 Ala Leu Gly Ala Gln Lys Glu Ala Ile Ser
```

```
                                              (*)               130
(1; con-            Pro Pro Asp Ala Ala Ser Ala Ala Pro Leu
tinued)

                                                              140
                   Arg Thr Ile Thr Ala Asp Thr Phe Arg Lys

                                                              150
                   Leu Phe Arg Val Tyr Ser Asn Phe Leu Arg

                                                              160
                   Gly Lys Leu Lys Leu Tyr Thr Gly Glu Ala

                   SH                     166
                   Cys Arg Thr Gly Asp(Arg)
```

```
(2)                         21  22  23  24  25  26
                           ···Glu Ala Glu Asn Ile Thr···
          EPO               GAG GCC GAG AAT ATC ACG
          Primer 1   3'-TC CGG CTC GTT TAG TCG-5'


(3)                         35  36  37  38  39  40
                           ···Leu Asn Glu Asn Ile Thr···
          EPO               TTG AAT GAG AAT ATC ACT
          Primer 2   3'-AC TTA CTC GTT TAG TGA-5'


(4)                         80  81  82  83  84  85  86
                           ···Leu Leu Val Asn Ser Ser Glu···
          EPO               CTG TTG GTC AAC TCT TCC CAG
          Primer 3   3' -C AAC CAG GTT AGA AGG G-5'
```

```
                                                 -27
                                 Met Gly Val His Glu Cys Pro

-20                                   -10                              -1
Ala Trp Leu Trp Leu Leu Leu Ser Leu Leu Ser Leu Pro Leu Gly Leu Pro Val Leu Gly

1                     SH          10                                  20
Ala Pro Pro Arg Leu Ile Cys Asp Ser Arg Val Leu Glu Arg Tyr Leu Leu Glu Ala Lys

            *                SH  30          SH                  *    40
Glu Ala Glu Asn Ile Thr Thr Gly Cys Ala Glu His Cys Ser Leu Asn Glu Asn Ile Thr

                                      50                              60
Val Pro Asp Thr Lys Val Asn Phe Tyr Ala Trp Lys Arg Met Glu Val Gly Gln Gln Ala

                                      70                              80
Val Glu Val Trp Gln Gly Leu Ala Leu Leu Ser Glu Ala Val Leu Arg Gly Gln Ala Leu

            *                         90                              100
Leu Val Asn Ser Ser Gln Pro Trp Glu Pro Leu Gln Leu His Val Asp Lys Ala Val Ser

                                      110                             120
Gly Leu Arg Ser Leu Thr Thr Leu Leu Arg Ala Leu Gly Ala Gln Lys Glu Ala Ile Ser

                (*)                   130                             140
Pro Pro Asp Ala Ala Ser Ala Ala Pro Leu Arg Thr Ile Thr Ala Asp Thr Phe Arg Lys

                                      150                             160
Leu Phe Arg Val Tyr Ser Asn Phe Leu Arg Gly Lys Leu Lys Leu Tyr Thr Gly Glu Ala

SH                  166
Cys Arg Thr Gly Asp(Arg)
```

# Fig. 1

```
             21  22  23  24  25  26
            ···Glu Ala Glu Asn Ile Thr···
EPO          GAG GCC GAG AAT ATC ACG
Primer 1  3'-TC CGG CTC GTT TAG TCG-5'


             35  36  37  38  39  40
            ···Leu Asn Glu Asn Ile Thr···
EPO          TTG AAT GAG AAT ATC ACT
Primer 2  3'-AC TTA CTC GTT TAG TGA-5'


             80  81  82  83  84  85  86
            ···Leu Leu Val Asn Ser Ser Glu···
EPO          CTG TTG GTC AAC TCT TCC CAG
Primer 3  3' -C AAC CAG GTT AGA AGG G-5'
```

# Fig. 2

Fig. 3

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | THE JOURNAL OF BIOLOGICAL CHEMISTRY, vol. 263, no. 33, 25th November 1988, pages 17516-17521, US, S. DUBE et al.: "Glycosilation at specific sites of erythropoietin is essential for biosynthesis, secretion, and biological function" * Page 17517, figure 1 * | 1-9 | C 07 K 13/00 C 12 N 15/16 |
| P,X | EP-A-0 357 804  (GENETICS INSTITUTE INC.) * Page 2, column 1, lines 25-55; column 2, lines 1-31; page 3, column 1, lines 46-58; column 2, lines 1-19 * | 1-9 | |
| X | PROC. NATL. ACAD. SCI. USA, vol. 81, May 1984, pages 2708-2712; S. LEE-HUANG: "Cloning and expression of human erythropoietin in cDNA in Escherichia coli" * Abstract * | 9 | |

TECHNICAL FIELDS SEARCHED (Int. Cl.5)

C 07 K
C 12 N

The present search report has been drawn up for all claims

| Place of search | Date of completion of search | Examiner |
|---|---|---|
| The Hague | 22 January 91 | NAUCHE S.A. |